# EUROPEAN PATENT APPLICATION

(11) **EP 3 456 326 A1**
(43) Date of publication of application: **20.03.2019**
(21) Application number: 17795442.7
(22) Date of filing: 28.04.2017
(51) Int. Cl.: A61K 31/357, A61K 31/366, A61K 31/541, A61P 3/04, A61P 3/10, A61P 3/06, A61P 1/16, A61P 5/50

(54) **ARTEMISININ ANALOG, AND USE, METHOD, AND COMPOSITION FOR PROMOTING LIPID CATABOLISM AND IMPROVING SUGAR METABOLISM**

(30) Priority: 10.05.2016 CN 201610305363
(71) Applicant: Fudan University, Shanghai 200032 (CN)
(72) Inventor: TANG, Qiqun, Shanghai 200032 (CN); DANG, Yongjun, Shanghai 200032 (CN); LU, Ping, Shanghai 200032 (CN); LI, Xi, Shanghai 200032 (CN); ZHANG, Fuchuang, Shanghai 200032 (CN); ZUO, Jianping, Shanghai 200032 (CN)
(74) Representative: Allaix, Roberto
(86) International application number: PCT/CN2017/082359
(87) International publication number: WO 2017/193824

(57) **Abstract**

An artemisinin analog as represented by formula (A) and application thereof in preparing a pharmaceutical product for promoting lipid catabolism and/or prevention or treatment of metabolism-related disease. The artemisinin analog promotes browning of white fat to achieve body weight reduction and improve metabolism, thereby providing therapeutic effects against hyperglycemia, insulin resistance, dyslipidemia, and/or fatty liver.

## Description

### TECHINICAL FIELD

The invention belongs to the field of biomedicine, and particularly relates to the use of artemether in preparing preparations for promoting lipolysis and improving glucose metabolism.

### BACKGROUND ART

Data show that obesity and its related metabolic diseases have become epidemic diseases that are causing trouble worldwide. According to data released by the World Health Organization (WHO) in 2015, the obesity rate of global population is 13%; and the obesity rate of adults is 6.9% in China. It is worried that the obesity rate of adolescents is higher and exhibits a trend of sharp increase, which means that obesity rate will continue to grow over the next few decades. Studies have shown that obesity can cause a series of metabolic diseases, such as type 2 diabetes, high blood pressure, fatty liver, etc., and even increase the incidence of certain tumors, which will bring great health burden and economic burden to the country and the people.

Studies have shown that adipose tissue is at the core position of the development and progression of obesity and plays a pivotal role in metabolic health. It is currently known that there are three kinds of adipose tissues in the human body, white fat, brown fat and beige fat. White adipose tissue stores energy in the form of triglyceride, while brown fat and beige fat consume energy through heat production. Beige fat is found in white fat and has a brown fat characteristic. Beige fat can be considered as a transitional form of white fat and brown fat in terms of phenotype. The phenomenon of the appearance of beige fat in white adipose tissue is called browning of white fat. Although beige fat and brown fat are in different parts and have different developmental origins, they exert the same thermogenic function, have the same potential in terms of energy consumption and are called thermogenic adipose tissue. Under physiological conditions, there are two conditions that activate thermogenic adipose tissue: cold exposure and dietary intake (especially high calorie foods). During cold exposure, the thermogenic adipose tissue is activated to maintain a constant body temperature; during dietary intake, activation of the thermogenic fat relieves the transient energy peak and maintains metabolic stability.

Thermogenic adipose tissue, its differentiation and development, functional regulation and clinical application are research hotspots in the field of metabolism in recent years. According to the population survey, individuals with active thermogenic adipose tissue are not prone to gain weight and are not susceptible to metabolism-related diseases such as type 2 diabetes. Some drugs that improve metabolism, such as berberine and salicyl salicylic acid, can achieve part of the effect by activating thermogenic adipose tissue. Similarly, in animal models, whether related genes are changed (knocked out or overexpressed), or drug stimulation (β-adrenergic receptor agonists, etc.) is carried out, after the activation of thermogenic brown fat, it will function to control body weight and improve metabolism. Therefore, in summary, beige adipose tissue is a thermogenic adipose tissue in human body, which has the potential to consume excess energy, and can be activated under certain conditions. Activation of thermogenic adipose tissue, whether to enhance brown fat function or to promote browning of white fat, can play a role in weight control and metabolism. To date, there have been neither compound or drug discovery that specifically target thermogenic adipose tissue to achieve improved metabolism, nor high-throughput screening for this target.

Thermogenic adipose tissue converts chemical energy stored in fatty acids into heat energy because it has a special molecule, uncoupling protein 1 (UCP1). UCP1, which is localized on the inner membrane of the mitochondria, can cut off the coupling of the electron transport chain and ATP synthesis and directly convert chemical energy into heat energy in the case of activation. UCP1 is found in thermogenic adipose tissue. Due to this unique tissue localization, UCP1 is not only a functional molecule of thermogenic adipose tissue, but also a phenotypic marker. In addition to UCP1, PGC1α, PRDM16 and cytochrome C are important molecules involved in the browning process.

Studies have reported that the occurrence of obesity and metabolic diseases is the result of a combination of living environment and genetic factors. High-fat diet-induced obese mice are animal models commonly used in laboratory to study the mechanism of the development and progression of obesity and metabolic diseases. Ob/ob mice have severe insulin resistance due to the lack of leptin gene, and have fatty liver since birth, and these metabolic disorders gradually aggravate with the growth of mice, eventually leading to premature death in mice. Due to the presence of these innate metabolic disorders, ob/ob mice are common animal models for laboratory studies of diabetes and insulin resistance. The commonly used cell models for in vitro study of fat cell differentiation and development are 3T3-L1 and C3H10T1/2, with the former simulating the process of differentiation of preadipocytes into mature adipocytes, and the latter reflecting the process of differentiation of mesenchymal stem cells into adipocytes after orientation. In addition to cell lines, the cell model for studying the development of fat also includes the Stromal Vascular Fraction (SVF). SVF is a primary preadipocyte, which can differentiate into white adipocyte after isolation, culture and certain induction, thereby mimicking the in vivo process of differentiation of preadipocytes into white fat in an in vitro environment.

The prior art has disclosed that artemether (ART) (Formula II) is an analogue of artemisinin (Formula I), which can efficiently and quickly kill erythrocyte-stage plasmodium. It is used in the treatment of chloroquine-resistant falciparum malaria and pernicious malaria, with rapid effect, good short-term efficacy and less toxicity and side effects. It is a first-line drug for the treatment of malaria and is widely used. Recent studies have shown that artemether also has an effect of killing tumor cells and immunomodulation, showing new potential in the treatment of tumors and autoimmune diseases such as systemic lupus erythematosus and rheumatoid arthritis.

The application potential of artemether in controlling body weight and improving metabolism has not yet been found. In addition to artemether, artemisinin-based drugs also include artesunate, dihydroartemisinin, arteether, SM905, artemiside, artemisone and SM934, among which dihydroartemisinin is a secondary metabolite of artemether and other artemisinin analogues in vivo.

Based on the research basis of the prior art, the inventor of the present application intend to provide novel use of artemether and artemisinin analogues in pharmaceuticals, in particular the use of artemether in the preparation of a preparation for promoting lipolysis and improving glucose metabolism.

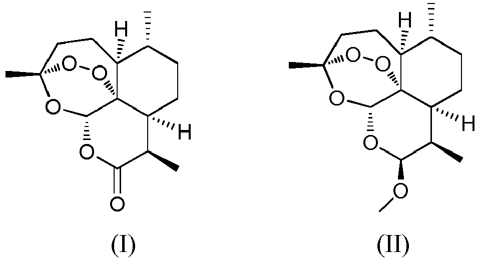

### SUMMARY OF THE INVENTION

Accordingly, the object of the present invention is to provide a novel use of artemether and artemisinin analogues in pharmaceuticals, in particular to the use of artemether in the preparation of a preparation for promoting lipolysis and improving glucose metabolism, especially to the use of artemether and artemisinin analogues in the preparation of a preparation for promoting browning of white fat, reducing body weight and improving glucose metabolism.

In order to achieve the above object, a first aspect of the present invention provides the use of an artemisinin analogue represented by the following general formula (A) in the preparation of a medicament for promoting lipolysis and/or preventing or treating a metabolism-related disease: 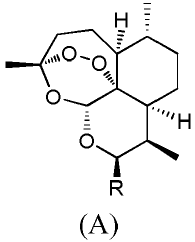 wherein, R is selected from hydroxyl, methoxy, ethoxy, butanedioic acid monoester group (succinate group), 2-aminoethoxy, 2R-3-*tert*-butylamino-2-hydroxypropoxy, thiomorpholinyl and 1,1-dioxidothiomorpholinyl.

A second aspect of the present invention provides a method for promoting lipolysis and/or preventing or treating a metabolism-related disease, said method comprises administering to a subject in need thereof a therapeutically effective amount of an artemisinin analogue represented by the following general formula (A): 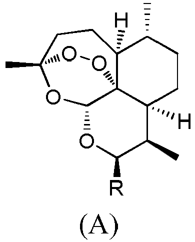 wherein, R is selected from hydroxyl, methoxy, ethoxy, butanedioic acid monoester group (succinate group), 2-aminoethoxy, 2R-3-*tert*-butylamino-2-hydroxypropoxy, thiomorpholinyl and 1,1-dioxidothiomorpholinyl.

Preferably, said administering a therapeutically effective amount of said artemisinin analogue is by oral or injection; more preferably, the administration dosage of said artemisinin analogue is 20mg/kg-100mg/kg, preferably 50mg/kg-100mg/kg for oral administration, and 10mg/kg-50mg/kg, preferably 20mg/kg-40mg/kg for injection, respectively.

A third aspect of the present invention provides an artemisinin analogue for promoting lipolysis and/or preventing or treating a metabolism-related disease, said artemisinin analogue is represented by the following general formula (A): 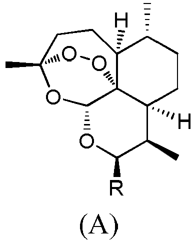 wherein, R is selected from hydroxyl, methoxy, ethoxy, butanedioic acid monoester group (succinate group), 2-aminoethoxy, 2R-3-*tert*-butylamino-2-hydroxypropoxy, thiomorpholinyl and 1,1-dioxidothiomorpholinyl.

Preferably, according to use, artemisinin analogue or method of any of the preceding aspects, said artemisinin analogue is artemether represented by formula (II):

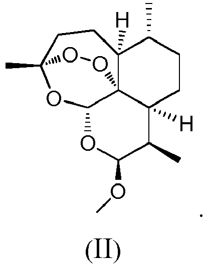

Preferably, according to use, artemisinin analogue or method of any of the preceding aspects, said promoting lipolysis comprises promoting browning of white fat.

Preferably, according to use, artemisinin analogue or method of any of the preceding aspects, said promoting lipolysis and/or preventing or treating a metabolism-related disease comprises controlling weight and improving metabolism.

Preferably, according to use, artemisinin analogue or method of any of the preceding aspects, said metabolism-related disease is hyperglycemia, insulin resistance, dyslipidemia, and/or fatty liver caused by obesity.

A fourth aspect of the present invention provides a pharmaceutical composition for promoting lipolysis and/or preventing or treating a metabolism-related disease, said pharmaceutical composition comprises: (1) the artemisinin analogue according to the third aspect of the present invention, and (2) a pharmaceutically acceptable carrier.

Preferably, the dosage form of said pharmaceutical composition for oral administration is selected from an oral suspension, a powder and a granule; and the dosage form of said pharmaceutical composition for injection is selected from an aqueous solution, an oil and a suspension.

The specific technical solutions of the present invention are further described below in conjunction with the concept of the present invention.

The structure of artemether of the present invention is as shown in formula (II).

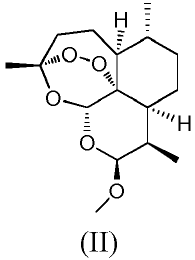

The present invention also relates to artemisinin analogues other than artemether which have the potential to promote browning of white fat. The artemisinin structure is represented by formula (I), and the artemisinin analogues are represented by formula (A).

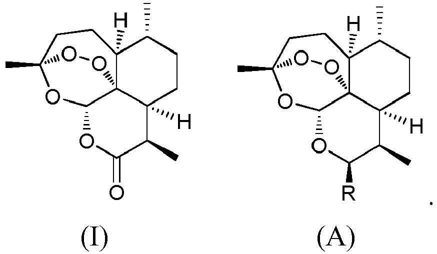

The artemisinin analogue represented by formula (A) is dihydroartemisinin, with R being hydroxyl; arteether, with R being ethoxy; artesunate, with R being butanedioic acid monoester group (succinate group); SM934, with R being 2-aminoethoxy; SM905, with R being 2R-3-*tert*-butylamino-2-hydroxypropoxy; artemiside, with R being thiomorpholinyl; artemisone, with R being 1,1-dioxidothiomorpholinyl.

The artemisinin analogues of the present invention have R groups as shown in the following table:

| | |
|---|---|
| Dihydroartemisinin | R=OH |
| Artemether | R=OMe |
| Artesunate | 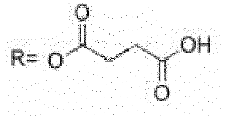 |
| SM934 | 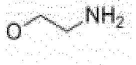 |
| SM905 | 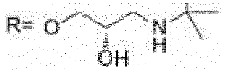 |
| Artemiside | 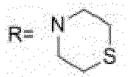 |
| Artemisone | 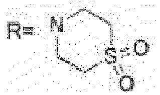 |

The invention demonstrates by in vivo and in vitro experiments that the artemisinin-based compounds have the effect of inducing browning of white fat, and play a role in reducing body weight, improving metabolism, maintaining glucose metabolism homeostasis, alleviating fatty liver, and improving symptoms associated with diabetes by promoting browning of white fat. The clinical application practice of the artemisinin-based drugs shows that no intolerable side effects have been found. In studies of rodent models, it has been reported that high concentrations of artemether would cause anorexia nervosa, but there is no clear evidence of dose and timing of administration. Therefore, overall, artemether has an ideal safety as a clinical drug.

### BRIEF DESCRIPTION OF THE DRAWINGS

Hereinafter, embodiments of the present invention will be described in detail with reference to the accompanying drawings, in which:

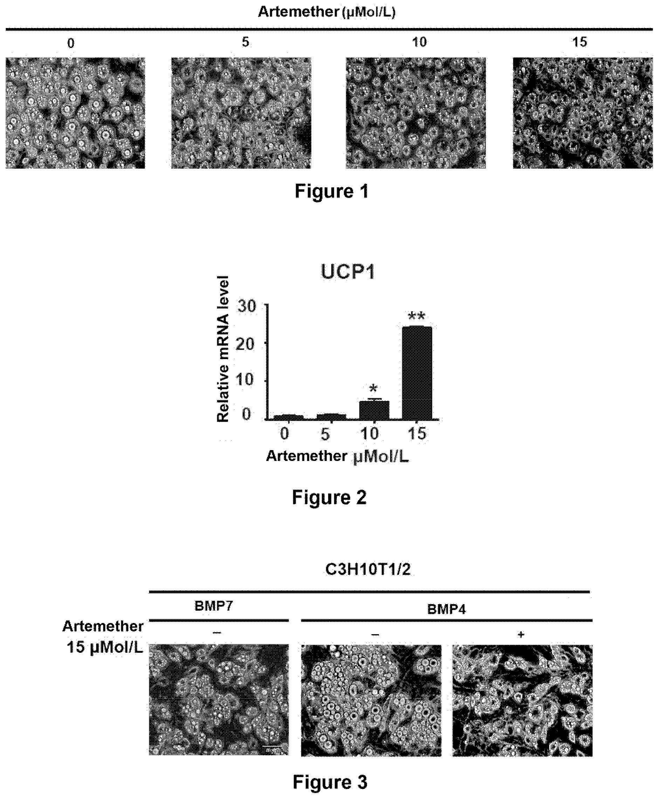

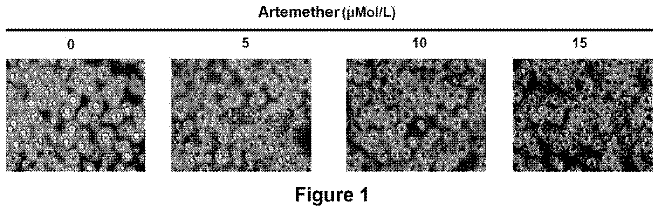

Figure 1 and Figure 2 show that artemether enabled 3T3-L1 to exhibit brown adipocyte-like characteristics. Among them, Figure 1 shows the morphological changes of 3T3-L1 cells treated with different concentrations of ART; Figure 2 shows the mRNA level of UCP1 after treating 3T3-L1 with different concentrations of ART. *p<0.05, **p<0.01.

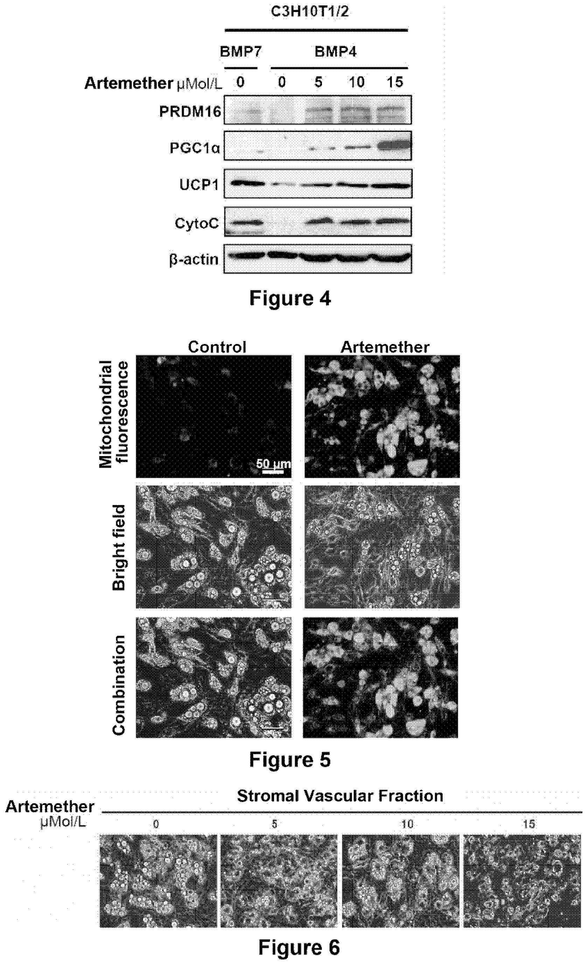

Figure 3-5 show that artemether enabled BMP4-directed C3H10T1/2 to exhibit brown fat-like morphological characteristics. Among them, Figure 3 shows the cell morphological characteristics of BMP7-directed C3H10T1/2, BMP4-directed C3H10T1/2, and BMP4-directed C3H10T1/2 treated with artemether; Figure 4 shows the protein levels of brown fat-related gene UCP1, PGC1a, PRDM16 and CytoC of BMP4-directed C3H10T1/2, BMP4-directed C3H10T1/2, and BMP4-directed C3H10T1/2 treated with different concentrations of artemether by Western Blot assay; Figure 5 shows the mitochondrial MitoTracker Green staining of BMP4-directed C3H10T1/2 after treatment with artemether (15 µMol/L).

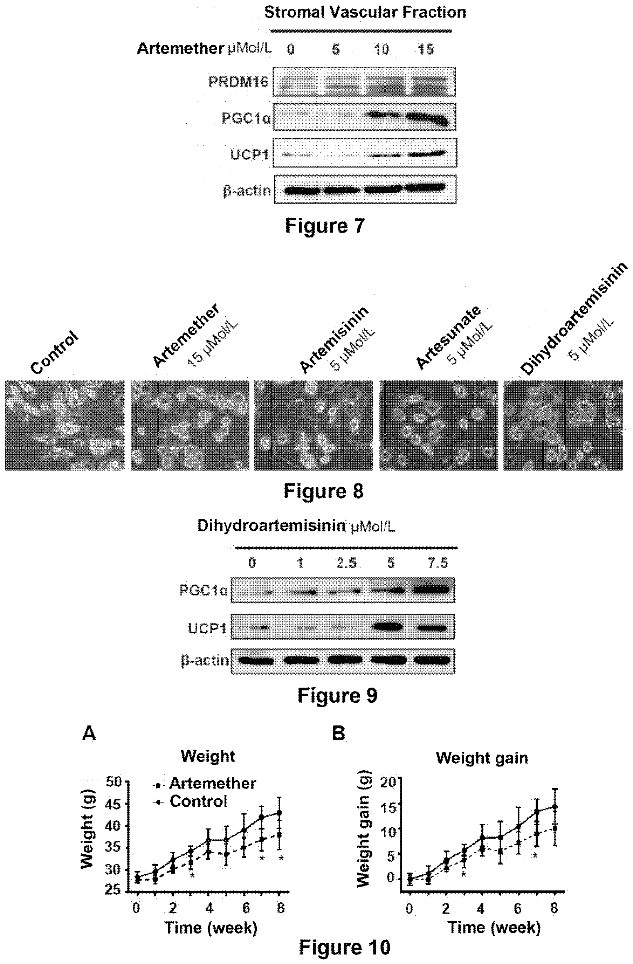

Figures 6-7 show that artemether enabled SVF primary preadipocyte to acquire brown fat-like morphological characteristics during differentiation. Among them, Figure 6 shows the cell morphological changes after treating SVF with different concentrations of artemether; Figure 7 shows the expression levels of brown fat-related gene PRDM16, PGC1α and UCP1 after treating SVF with different concentrations of artemether.
Figures 8-9 illustrate the effect of artemisinin analogues on promoting browning. Among them, Figure 8 shows the cell morphology of BMP4-directed C3H10T1/2 treated with artemisinin and its analogues such as artemether, artesunate and dihydroartemisinin; Figure 9 shows the expression levels of brown fat-related gene after treating BMP4-directed C3H0T1/2 with different concentrations of dihydroartemisinin.
Figure 10 shows that artemether inhibited weight gain induced by a high fat diet. Among them, Figure 10A shows the change in weight of mice subcutaneously injected with artemether under a high-fat diet condition, *p<0.05; Figure 10B shows the increase of weight in mice subcutaneously injected with artemether under a high-fat diet condition, *p<0.05.

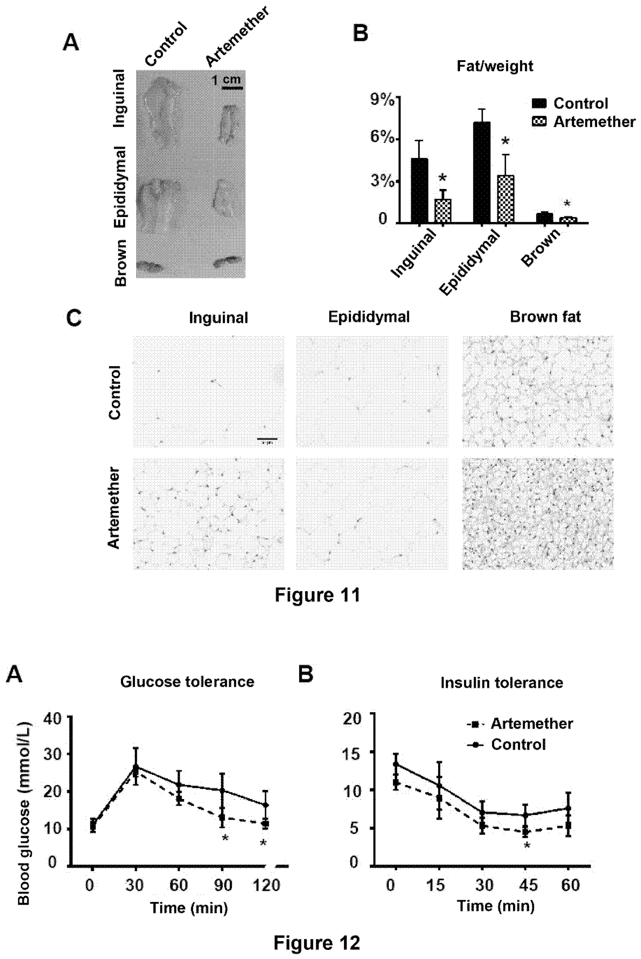

Figure 11 shows that subcutaneous injection of artemether reduced adipose tissue in high fat-fed mice. Among them, Figure 11A shows the volume of brown fat of inguinal fat, epididymal fat, and interscapular region of mice subcutaneously injected with artemether and mice in control group; Figure 11B shows the ratio of the weight of brown fat of inguinal fat, epididymal fat, and interscapular region of mice subcutaneously injected with artemether and mice in control group to the weight of mice, *p<0.05; Figure 11C shows the morphology of brown adipocytes of inguinal fat, epididymal fat, and interscapular region of mice subcutaneously injected with artemether and mice in control group.
Figure 12 shows that subcutaneous injection of artemether improved glucose metabolism in high fat-induced obese mice. Among them, Figure 12A shows glucose tolerance curve of mice subcutaneously injected with artemether and mice in control group after eight weeks of high-fat diet feeding, *p<0.05; Figure 12B shows the glucose tolerance curve of mice injected subcutaneously with artemether and mice in control group after eight weeks of high-fat diet feeding, *p<0.05.

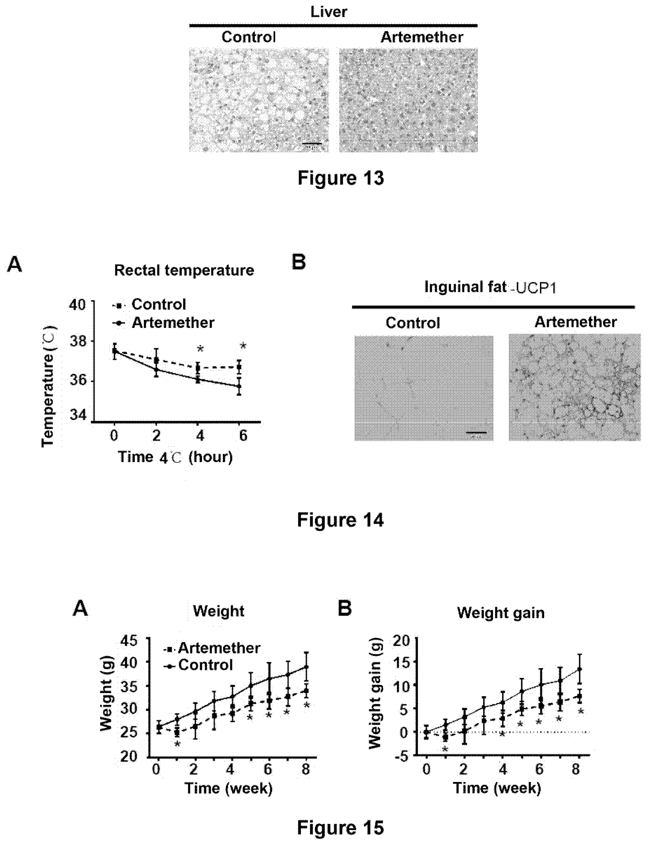

Figure 13 shows that artemether inhibited fatty liver induced by a high-fat diet. It shows the liver cell morphology of mice injected subcutaneously with artemether and mice in control group after eight weeks of high-fat diet feeding.
Figure 14 shows that subcutaneous injection of artemether induced browning of subcutaneous fat in high fat-fed mice. Among them, Figure 14A shows the change in body temperature of high fat-fed mice in an environment of 4 °C after eight weeks of administration, *p<0.05; Figure 14B shows the expression of UCP1 in inguinal adipose tissue of high fat-fed mice after exposure to 4 °C for 6 hours after eight weeks of administration.
Figure 15 shows that intraperitoneal injection of artemether inhibited weight gain induced by a high fat diet. Among them, Figure 15A shows the change in weight of mice injected intraperitoneally with artemether under a high-fat diet condition, *p<0.05; Figure 15B shows the increase of weight in mice injected intraperitoneally with artemether under a high-fat diet condition, *p<0.05.

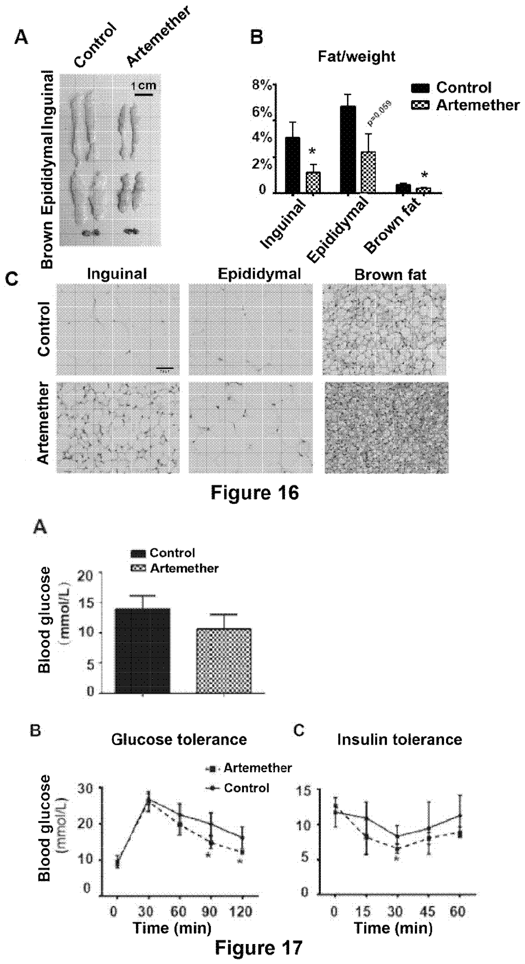

Figure 16 shows that intraperitoneal injection of artemether significantly reduced adipose tissue in high fat-fed mice. Among them, Figure 16A shows the volume of brown fat of inguinal fat, epididymal fat, and interscapular region of mice injected intraperitoneally with artemether and mice in control group; Figure 16B shows the ratio of the weight of brown fat of inguinal fat, epididymal fat, and interscapular region of mice injected intraperitoneally with artemether and mice in control group to the weight of mice, *p<0.05; Figure 16C shows the morphology of brown adipocytes of inguinal fat, epididymal fat, and interscapular region of mice injected intraperitoneally with artemether and mice in control group.
Figure 17 shows that intraperitoneal injection of artemether improved glucose metabolism in high fat-induced obese mice. Among them, Figure 17A shows the random blood glucose of mice injected intraperitoneally with artemether and mice in control group after eight weeks of high-fat diet feeding, *p<0.05; Figure 17B shows the glucose tolerance test results of mice injected intraperitoneally with artemether and mice in control group after eight weeks of high-fat diet feeding, *p<0.05; Figure 17C shows the insulin tolerance of mice injected intraperitoneally with artemether and mice in control group after eight weeks of high-fat diet feeding, *p<0.05.

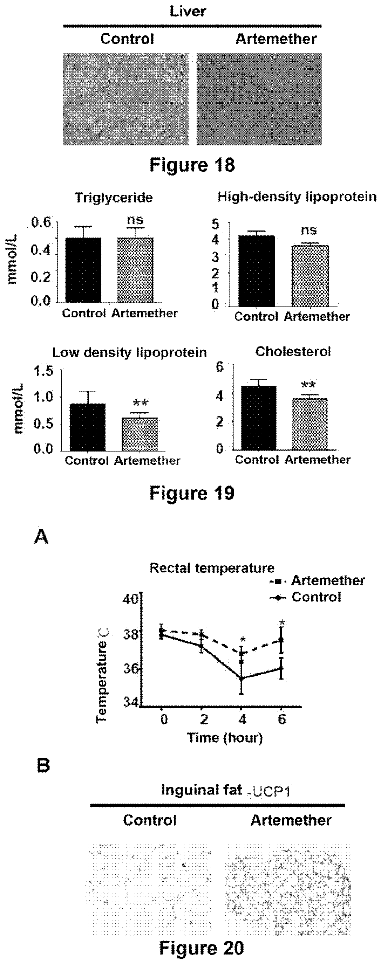

Figure 18 shows the histological morphology of liver cells of mice injected intraperitoneally with artemether and mice in control group after eight weeks of high-fat diet feeding.
Figure 19 shows after eight weeks of high-fat diet feeding, changes in lipid metabolism related indexes in serum of mice injected intraperitoneally with artemether compared with mice in control group, *p<0.05, **p<0.01.
Figure 20 shows that intraperitoneal injection of artemether induced browning of subcutaneous fat in high fat-fed mice. Among them, Figure 20A shows the change in body temperature of high fat-fed mice in an environment of 4 °C after eight weeks of intraperitoneal injection of artemether, *p<0.05; Figure 20B shows the expression of UCP1 in inguinal adipose tissue of high fat-fed mice after exposure to 4 °C for 6 hours after eight weeks of intraperitoneal injection of artemether.

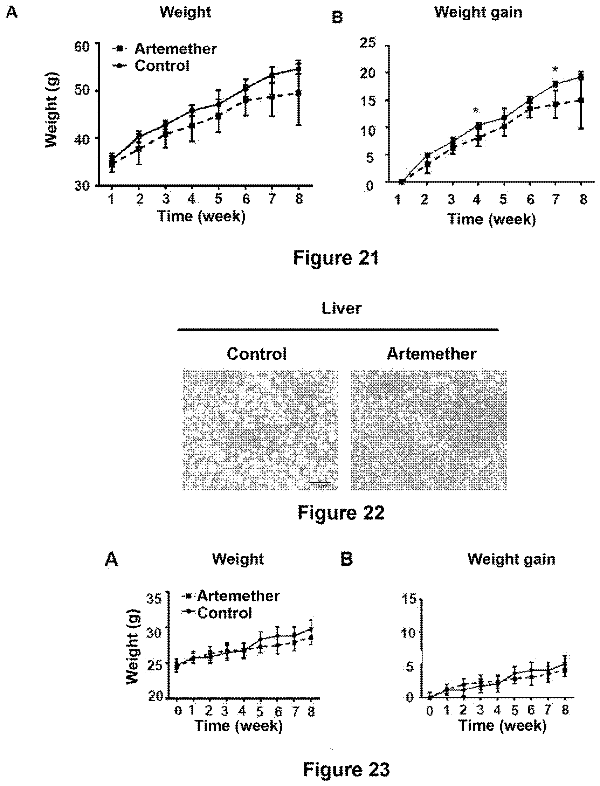

Figure 21 shows that artemether inhibited weight gain in diabetic ob/ob mice. Among them, Figure 21A shows the change in weight of ob/ob mice injected intraperitoneally with artemether for eight weeks; FIG. 21B shows the change in weight gain of ob/ob mice injected intraperitoneally with artemether for eight weeks, *p< 0.05.
Figure 22 shows that artemether improved fatty liver in diabetic ob/ob mice, and it shows the results of HE staining of liver sections of ob/ob mice after eight weeks of intraperitoneal injection of artemether.
Figure 23 shows that intraperitoneal injection of artemether did not affect growth of healthy mice. Among them, Figure 23A shows the weight of normal diet-fed healthy mice injected intraperitoneally with artemether for eight weeks in administration group and control group; Figure 23B shows the weight gain of normal diet-fed healthy mice injected intraperitoneally with artemether for eight weeks in administration group and control group.

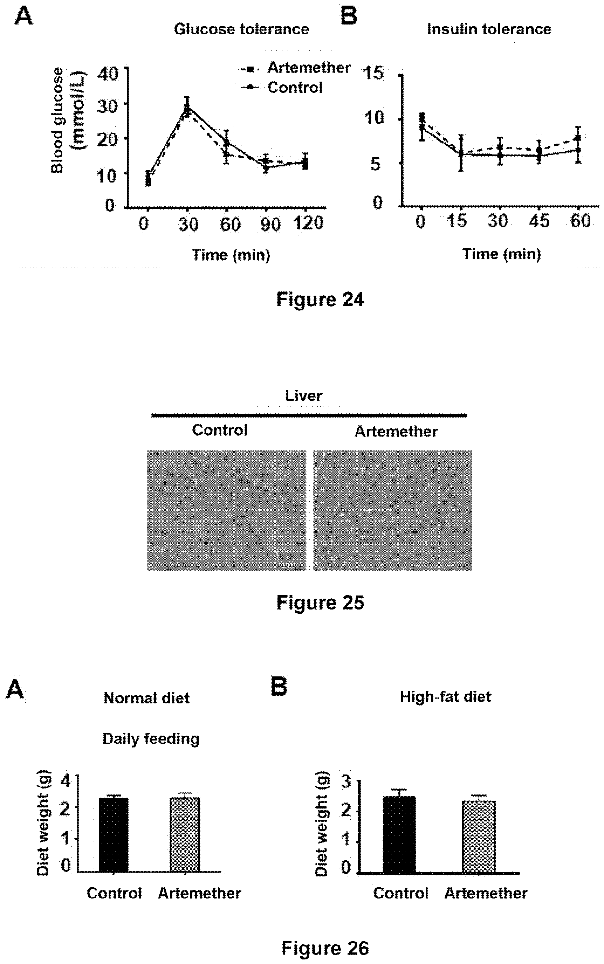

Figure 24 shows that intraperitoneal injection of artemether did not affect glucose metabolism of healthy mice. Among them, Figure 24A shows glucose tolerance of healthy lean mice injected intraperitoneally with artemether for eight weeks; Figure 24B shows glucose insulin tolerance of healthy lean mice injected intraperitoneally with artemether for eight weeks.
Figure 25 shows that intraperitoneal injection of 20 mg/kg weight of artemether in mice did not cause liver damage, and it shows the results of HE staining of liver tissue sections of healthy lean mice after eight weeks of intraperitoneal injection of artemether.
Figure 26 shows that artemether did not alter the food intake of mice. Among them, Figure 26A shows daily food intake per mouse of normal diet-fed lean mice injected intraperitoneally with artemether for eight weeks; Figure 26B shows daily diet consumption per mouse of high-fat diet-fed obese mice injected intraperitoneally with artemether for eight weeks.

### BEST MODE FOR CARRYING OUT THE INVENTION

### EXAMPLE 1. Artemether induced browning of 3T3-L1 white prea dipocyte during differentiation

### 1.1 Materials

1.1.1 3T3-L1 white preadipocytes
1.1.2 DMEM medium (Gibco)
1.1.3 Fetal bovine serum (Gibco)
1.1.4 Calf serum (TBD)
1.1.5 Biotin (Sigma)
1.1.6 Penicillin and streptomycin
1.1.7 Artemether (Chengdu Pufei De Biotech Co., Ltd.) 1.1.8 Trizol (Life Technology)
1.1.9 Reverse transcription kit (ThermoScientific)
1.1.10 Power SYBR Green Master Mix (ThermoScientific)

### 1.2 Equipment

1.2.1 Constant temperature sterile incubator (Thermo Scientific Forma Series II Water Jacket)
1.2.2 Real-time quantitative PCR instrument (Applied Biotech 7500 real time PCR System)
1.2.3 Microscope (Olympus IX711)
1.2.5 Refrigerated centrifuge (Eppendorf Centrifuge 5427R)
1.2.6 Nucleic acid quantitative instrument (Colibri)

### 1.3 Solution

1.3.1 Medium
3T3-L1 maintenance medium

| DMEM | Up to 500 ml |
|---|---|
| Calf serum | 10% |
| Biotin | 8mg/ml |
| Penicillin | 100U/ml |
| Streptomycin | 100U/ml |

3T3-L1 induced differentiation medium

| DMEM | Up to 500 ml |
|---|---|
| Fetal bovine serum | 10% |
| Biotin | 8mg/ml |
| Penicillin | 100U/ml |
| Streptomycin | 100U/ml |

1.3.2 Artemether stock solution was dissolved in DMSO at a storage concentration of 10 mMol/L.
1.3.3 Biotin: 160mg biotin and 80mg calcium pantothenate were dissolved in 200ml deionized water, and heated to dissolve. The solution was filtered and sterilized through 0.22µm filter membrane.
1.3.4 3-Isobutyl-1-methylxanthine (50mMol/L): 1.15g 3-isobutyl-1-methylxanthine was dissolved in 100ml 0.2M potassium hydroxide, and the solution was filtered and sterilized through 0.22µm filter membrane.
1.3.5 Dexamethasone (1mMol/L): 0.039g dexamethasone was dissolved in 100ml water, and the solution was filtered and sterilized through 0.22µm filter membrane.
1.3.6 Insulin (1mg/ml): 0.1g insulin was dissolved in 100ml water, a few drops of hydrochloric acid were added to dissolve the insulin, and the solution was filtered and sterilized through 0.22 µm filter membrane.

### 1.4 Method

### 1.4.1 Culture and induced differentiation of white preadipocyte 3T3-L1

2.5×10⁵ 3T3-L1 white preadipocytes were seeded in a 3.5cm cell culture dish, and cultured for 3 days in a maintenance medium. Cells proliferated to contact inhibition. 48 hours after the contact inhibition was reached, the medium was changed to induced differentiation medium, and the induced differentiation mixture including 0.5 mMol/L 3-isobutyl-1-methylxanthine (Mix), 1 µMol/L dexamethasone (Dex) and 1µg/ml insulin (Insulin) was added. The induced differentiation mixture is referred to as MDI for short. The day of adding MDI was defined as Day 0 of differentiation. After 2 days of culture in the above medium, fresh induced differentiation medium was replaced, and 1 µg/ml insulin was added to the medium. After another 2 days of culture, the fresh induced differentiation medium was replaced, and the medium was changed every other day until the culture was differentiated to Day 8.

### 1.4.2 Compound treatment

Artemether was stored in DMSO at a storage concentration of 10mMol/L. While MDI was added on Day 0 of differentiation, artemether (5, 10 or 15µMol/L) was added for treatment. Hereafter, each time the medium were changed, the same concentration of artemether was added until 3T3-L1 was differentiated to mature.

### 1.4.3 Extraction of cell total RNA from 3T3-L1 and reverse transcription into cDNA

3T3-L1 differentiated to mature was induced in a 3.5 cm culture dish, with the method of differentiation referring to Method 1.4.1. After washing with PBS, the cells were fully lysed with 1 ml Trizol, thoroughly mixed, and 200 µl chloroform was added. After violently shaking, the mixtre was allowed to stand at room temperature for 15 min, and centrifuged at 4 °C, with the centrifuge condition being at 12000 g for 10 min. At this time, the lysate was separated into three layers due to the chromatographic action. The upper aqueous phase solution was carefully pipetted into a new centrifuge tube, and 500 µl isopropanol was added. After gently mixing, the mixture was allowed to stand for 10 min at room temperature, centrifuged at 12000 g for 10 min at 4 °C. The supernatant was discarded and the milky white translucent precipitate was seen at the bottom of the tube. The precipitate was washed 3 times with 75% ethanol diluted with DEPC water, centrifuged at 7500 g for 5 min at 4 °C. The supernatant was discarded and air dried at room temperature. The precipitate was dissolved in 60 µl DEPC water, and quantified by Colibri Tepertec and stored at -80 °C.

The corresponding cDNA was synthesized by reverse transcription from 1 µg RNA, and the reverse transcription kit was Thermo Scientific Revert Aid First Strand cDNA Synthesis Kit. The synthesis system was as follows:

| | |
|---|---|
| M-MLV Reaction Buffer | 4µl |
| 10 mM dNTP | 2µl |
| RNase inhibitor | 1µl |
| M-MLV reverse transcriptase | 1µl |
| DEPC water | Up to 20µl |

The synthesis conditions were as follows:

| | |
|---|---|
| 65 °C | 5min |

| Cooling on ice | |
|---|---|
| 25 °C | 5min |
| 42 °C | 60min |
| 70 °C | 5min |

The synthesized cDNA was stored in a refrigerator at -20 °C.

### 1.4.4 Real time qPCR

Reaction system:

| | |
|---|---|
| CYBR Green Master Mix | 15µl |
| Upstream primer | 0.5µl |
| Downstream primer | 0.5µl |
| RNA template | 0.5µl |
| ddH₂O | 14.5µl |

The above system had a total volume of 30 µl and was divided into three wells, each of which had a volume of 10 µl.

Reaction conditions:

| | | | |
|---|---|---|---|
| 50°C | 2min | | |
| 95°C | 10min | | |
| 95°C | 15sec | 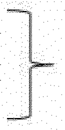 | 40 cycles |
| 60°C | 30sec | | |
| 95°C | 30sec | | |
| 60°C | 15sec | | |

The forward primer sequence for amplification of 18S was GTAACCCGTTGAACCCCATT; the reverse primer sequence was CCATCCAATCGGTAGTAGCG.

The forward primer sequence for amplification of UCP1 was AGGCTTCCAGTACCATTAGGT; the reverse primer was CTGAGTGAGGCAAAGCTGATTT.

The relative amount of mRNA was calculated according to the ΔΔCT method, with 18S being used as an internal reference. The level of control mRNA was set to 1, and each treatment group was compared with the control group to obtain a relative amount.

### 1.4.5 Data analysis

Data difference was analyzed by Student T test method, and p<0.05 indicates statistical difference.

### 1.5 Results

### 1.5.1 Artemether enabled 3T3-L1 to exhibit brown adipocyte-like characteristics

Cell morphology was observed under an Olympus 1X711 microscope and recorded. As shown in Figure 1, 3T3-L1 in control group showed typical white adipocyte characteristics, the cell volume was big, the lipid droplets were big and round, and the number of lipid droplets was less. 3T3-L1 treated with artemether (ART) exhibited typical brown adipocyte-like characteristics, the cell volume was smaller and the lipid droplets were small and dense. And this change had a concentration-dependent tendency. As the concentration of artemether increased, the change of 3T3-L1 adipocytes was more obvious.

### 1.5.2 Artemether up-regulated the expression of UCP1 in 3T3-L1

The total RNA of 3T3-L1 was extracted and reverse transcribed into cDNA. The mRNA level of UCP1 was detected by Real time qPCR. Compared with the control group, the expression of UCP1 in ART-treated 3T3-L1 was significantly up-regulated and exhibited a concentration-dependent tendency.

3T3-L1 is a classic white preadipocyte. Artemether enabled 3T3-L1 to acquire brown fat-like characteristics during differentiation. It not only had the morphological characteristics of brown fat, but also exhibited the expression of marker of brown fat, UCP1.

### EXAMPLE 2. Artemether induced C3H10T1/2 mesenchymal stem cells to exhibit brown fat-like characteristics during differentiation into adipocytes

### 2.1 Materials

2.1.1 C3H10T1/2 mesenchymal stem cell line
2.1.2 DMEM medium (Gibco)
2.1.3 Fetal bovine serum (Gibco)
2.1.4 Calf serum (TBD)
2.1.5 Biotin (Sigma)
2.1.6 Penicillin and streptomycin
2.1.7 Artemether (Chengdu Pufei De Biotech Co., Ltd.)
2.1.8 BMP4 (R&D)
2.1.9 BMP7 (Prospec)
2.1.8 PVDF membrane (Millipore Immunobilon)
2.1.9 Chemiluminescent liquid numECL ultra (NCM Biotech Co., Ltd.)
2.1.10 MitoTracker Green (Life Technology)
2.1.11 Other unmentioned reagents were purchased from Sigma.

### 2.2 Equipment

2.2.1 Constant temperature sterile incubator (Thermo Scientific Forma Series II Water Jacket)
2.2.2 Microscope (Olympus IX711)
2.2.3 Electrophoresis apparatus (BioRad PowerPac HC)
2.2.4 Automatic chemiluminescence apparatus (ImageQuant LAS4000 Mini)

### 2.3 Solution

### 2.3.1 Medium

C3H10T1/2 maintenance medium

| DMEM | Up to 500 ml |
|---|---|
| Calf serum | 10% |
| Biotin | 8mg/ml |
| Penicillin | 100U/ml |
| Streptomycin | 100U/ml |

C3H10T1/2 induced differentiation medium

| DMEM | Up to 500 ml |
|---|---|
| Fetal bovine serum | 10% |
| Biotin | 8mg/ml |
| Penicillin | 100U/ml |
| Streptomycin | 100U/ml |

### 2.3.2 Artemether stock solution was dissolved in DMSO at a storage concentration of 10 mMol/L.

### 2.3.3 Biotin

160mg biotin and 80mg calcium pantothenate were dissolved in 200ml deionized water, and heated to dissolve. The solution was filtered and sterilized through 0.22µm filter membrane.

### 2.3.4 3-isobutyl-1-methylxanthine (50mMol/L)

1.15g 3-Isobutyl-1-methylxanthine was dissolved in 100ml 0.2M potassium hydroxide, and the solution was filtered and sterilized through 0.22µm filter membrane.

### 2.3.5 Dexamethasone (1mMol/L)

0.039g Dexamethasone was dissolved in 100ml water, and the solution was filtered and sterilized through 0.22µm filter membrane.

### 2.3.6 Insulin (1mg/ml)

0.1g Insulin was dissolved in 100ml water, a few drops of hydrochloric acid were added to dissolve the insulin, and the solution was filtered and sterilized through 0.22 µm filter membrane.

2.3.7 Lysis buffer for cell protein extraction

| | |
|---|---|
| Tris-HCL (pH6.8) | 50mMol/L |
| SDS | 2% (w/v) |
| Glycerol | 10% (w/v) |
| NaF | 10mMol/L |
| Na3VO4 | 100mMol/L |

### 2.4 Method

### 2.4.1 Culture and differentiation of C3H10T1/2

7.5×10⁵ C3H10T1/2 were seeded in a 3.5 cm cell culture dish, and cultured for 3-5 days in a maintenance medium. On the next day of seeding, BMP4 (at a final concentration of 20 ng/ml) was added to the medium to allow the cells to proliferate to contact inhibition. Two days after the contact inhibition was reached, the medium was changed to induced differentiation medium, and the induced mixture (0.5mMol/L Mix, 1µMol/L Dex, 1ng/ml insulin, 100 nMol/L indometacin, InM T3) was added at the same time. This day was defined as Day 0 of differentiation. After 2 days of induction, fresh induced differentiation medium was replaced, and 1 ng/ml insulin and 1nMol/L T3 were added. After another 2 days of culture, the fresh medium was replaced every other day until the culture was differentiated to Day 8 at which time the cells were mature. At this time, the differentiated and mature cells were white adipocytes.

7.5×10⁵ C3H10T1/2 were seeded in a 3.5 cm cell culture dish, and cultured for 3-5 days in a maintenance medium. On the next day of seeding, BMP7 (at a final concentration of 100 ng/ml) was added to the medium to allow the cells to proliferate to contact inhibition. Two days after the contact inhibition was reached, the medium was changed to induced differentiation medium, and the induced mixture (0.5mM Mix, 1µM Dex, 1ng/ml insulin, 100 nM indometacin, InM T3) was added at the same time. This day was defined as Day 0 of differentiation. After 2 days of induction, fresh induced differentiation medium was replaced, and 1 ng/ml insulin and 1nM T3 were added. After another 2 days of culture, the fresh medium was replaced every other day until the culture was differentiated to Day 8 at which time the cells were mature. At this time, the cells differentiated to mature were brown adipocytes.

### 2.4.2 Artemether treatment

On Day 0 of differentiation, that is, when the induced differentiation mixture was added, the compound was added to the medium at a storage concentration of 10mMol/L, and the final concentrations were 5µMοl/L, 10µMol/L and 15µMol/L, respectively. When the fresh medium was replaced, the corresponding compound was supplemented, and the compound was always with the cells until the cells differentiated to mature.

### 2.4.3 Extraction of cell total protein

C3H10T1/2 differentiated to mature was induced in a 3.5 cm culture dish, with the method of differentiation referring to Method 2.4.1. 300µl Trizol was added to fully lyse the cells, and the lysate was transferred into a 1.5 ml centrifuge tube and heated at 100 °C for 10 min. The lysate was centrifuged at 13000g for 10min at 4 °C, the lipid in the upper layer was removed, and the clarified lysate in the middle layer was transferred into a new centrifuge tube. 2.5 µl lysate was taken and quantified by BCA method, and 200 µl lysate was mixed with 50 µl loading buffer, heated at 100 °C for 10 min, and stored at -20 °C until use.

### 2.4.4 Western blot

30µg C3H0T1/2 protein was loaded onto 10% polyacrylamide gel, and electrophoresis was carried out, with electrophoresis being carried out on base gel at 80V, and on separating gel at 100V, until the target band was separated. The protein was transferred to a PVDF membrane in an ice bath at 100 V for 100 min. After the transfer was completed, it was blocked for 1 hr in 5% skim milk prepared with TBST (TBS buffer + 0.5% Tween), and incubated with specific antibodies overnight at 4 °C. The next day, the PVDF membrane was washed three times with TBST at room temperature for 10 min, incubated with the corresponding secondary antibody at room temperature for 1 hr, then washed three times with TBST at the same condition as that of primary antibody, incubated with chemiluminescent reagent at room temperature for 1 min, detected with ImageQuant LAS4000 Mini Automatic chemiluminescence apparatus and exposed, and the results were recorded. Catalog number of antibody and dilution ratio of antibody are shown in the table below:

| Antibody | Catalog number | Dilution ratio |
|---|---|---|
| β-Actin | Proteintech HRP-60008 | 1: 10000 |
| PRDM16 | Abcam Ab 106410 | 1: 200 |
| PGC1α | Abcam Ab54481 | 1: 400 |
| UCP1 | Abcam Ab10983 | 1: 200 |
| CytoC | Proteintech 10993-1-AP | 1: 1000 |
| Secondary antibody | Jackson Immunology 111-035-003 | 1: 10000 |

### 2.4.4 Labeling of active mitochondria with MitoTracker Green

C3H10T1/2 differentiated to mature was induced in a 3.5cm culture dish.

The medium was removed and 1ml PBS was added. MitoTraker Green was added to PBS at a final concentration of 100ng/ml. The mixture was incubated at 37 °C for 10 min, and the green fluorescence was observed under a fluorescence microscope. Representative field of view was captured and recorded.

### 2.5 Results

### 2.5.1 Artemether enabled BMP4-directed C3H10T1/2 to exhibit brown fat-like morphological characteristics

C3H10T1/2 is a mesenchymal stem cell with multipotential differentiation potential, with BMP4-directed C3H10T1/2 differentiating into white adipocyte, BMP7-directed C3H10T1/2 differentiating into brown adipocyte. During differentiation of BMP4-directed C3H10T1/2, artemether (15µMol/L) was added simultaneously for treatment. Cell morphology was observed, BMP7-directed C3H10T1/2 had typical brown adipocyte characteristics, the cell was small, the lipid droplets were small with a large quantity; BMP4-directed C3H10T1/2 had typical white adipocyte characteristics, the cell was big, the lipid droplets were big with a small quantity; during differentiation of BMP4-directed C3H10T1/2, artemether (15µMol/L) was added simultaneously for treatment, and the cells exhibited brown fat-like characteristics, which was similar to BMP7-directed cell morphology (as shown in Figure 3).

### 2.5.2 Artemether up-regulated the expression of brown fat-related genes after BMP4-directed C3H10T1/2 cells differentiated to mature

BMP7-directed C3H10T1/2 was used as a positive control. BMP4-directed C3H10T1/2 was treated with different concentrations of artemether (5, 10, 15 µMol/L), and the protein level of brown fat-related genes was detected by Western blot with β-actin as an internal reference. The results showed that artemether increased the protein levels of the brown fat-related genes UCP1, PGC1a, PRDM16 and CytoC in BMP4-directed C3H10T1/2 cells, and the increase had a concentration-dependent tendency (as shown in Figure 4).

### 2.5.3 Artemether enhanced mitochondrial biosynthesis of BMP4-directed C3H10T1/2

The mitochondria of BMP4-directed C3H10T1/2 were labeled with MitoTracker Green, and the mitochondrial biosynthesis of C3H10T1/2 after treatment with artemether (15 µMol/L) was observed to be significantly enhanced (as shown in Figure 5).

BMP4 enabled C3H10T1/2 mesenchymal stem cells to differentiate into cells with white fat characteristics. After treatment with artemether, BMP4-directed C3H10T1/2 differentiated to mature and exhibited brown adipocyte-like characteristics, which included the following three aspects: cell morphology, related gene expression and mitochondrial biosynthesis enhancement.

### EXAMPLE 3. Artemether enabled Stromal Vascular Fraction (SVF) to acquire brown adipocyte-like characteristics during differentiation.

### 3.1 Materials

3.1.1 Male C57BL/6J mice (4-6 week, Shanghai Slike experimental animal Co., Ltd.)
3.1.2 DMEM/F12 (1:1) medium (Gibco)
3.1.3 Fetal bovine serum (Gibco)
3.1.4 Biotin (Sigma)
3.1.5 Penicillin and streptomycin
3.1.6 Artemether (Chengdu Pufei De Biotech Co., Ltd.)
3.1.7 PVDF membrane (Millipore Immunobilon)
3.1.8 Chemiluminescent reagent numECL ultra (NCM Biotech Co., Ltd.)
3.1.10 Other unmentioned reagents were purchased from Sigma.

### 3.2 Equipment

3.2.1 Constant temperature sterile incubator (Thermo Scientific Forma Series II Water Jacket)
3.2.2 Microscope (Olympus IX711)
3.2.3 Electrophoresis apparatus (BioRad PowerPac HC)
3.2.4 Automatic chemiluminescence apparatus (ImageQuant LAS4000 Mini)

### 3.3 Solution

### 3.3.1 SVF medium (differentiation and culture

| DMEM/F12 (1:1) | Up to 500 ml |
|---|---|
| Fetal bovine serum | 10% |
| Biotin | 8mg/ml |
| Penicillin | 100U/ml |
| Streptomycin | 100U/ml |

### 3.3.2 Lysis buffer for cell protein extraction cell

| | |
|---|---|
| Tris-HCL (pH6.8) | 50mMol/L |
| SDS | 2% (w/v) |
| Glycerol | 10% (w/v) |
| NaF | 10mMol/L |
| Na₃VO₄ | 100mMol/L |

### 3.4 Method

### 3.4.1 Separation, culture and differentiation of SVF

Inguinal fat of male mice was cut into pieces, treated with collagenase at 37 °C for 15 min, and centrifuged at 250 rpm for 10 min. The pellet was resuspended in F12/DMEM medium. 2.5×10⁵ cells were taken and seeded in a 3.5 cm culture dish, which was pretreated with 1 ml polylysine for 30 min. Two days after cell contact inhibition (defined as Day 0 of differentiation), induction was performed according to adipocyte differentiation protocol as follows: two days after induction with F12/DMEM comprising 10% fetal bovine serum, 0.5mMol/L Mix, 1µMol/L Dex, 5µg/ml insulin, and 1µMol/L rosiglitazone; the culture was continued for 2 days with DMEM/F12 comprising 10% fetal bovine serum, 5µg/ml insulin and 1µMol/L rosiglitazone; thereafter, DMEM/F12 medium containing 10% fetal bovine serum was replaced every other day until the cells differentiated to Day 8.

### 3.4.2 Treatment of SVF with artemether

On Day 0 of differentiation, SVF was treated with different concentrations of artemether (5, 10, 15 µMol/L), then fresh medium was replaced, and the corresponding concentration of artemether was added until the cells differentiated to mature.

### 3.4.3 Extraction and Western Blot of SVF cell total protein

The method for extracting total SVF protein was the same as 2.4.3, Western blot method and catalog number of antibody was the same as 2.4.4.

### 3.5 Results

### 3.5.1 Artemether enabled SVF primary preadipocyte to acquire brown fat-like morphological characteristics during differentiation

During the differentiation of SVF primary preadipocyte, cells were treated with different concentrations of artemether until SVF differentiated to mature. Compared with standard differentiated control cells, artemether treated cells were smaller in size, with characteristics of multi-cavity small lipid droplets. And this change had a concentration-dependent tendency (as shown in Figure 6).

### 3.5.2 Artemether upregulated the expression of brown fat-related genes during SVF differentiation

The expression levels of brown fat-related genes in SVF cells treated with artemether were detected by Western Blot. The expression levels of PRDM16, PGC1α and UCP1 were found to gradually increase with the increase of artemether concentration. As shown in Figure 7. SVF is a primary preadipocyte, under the action of artemether, SVF differentiated into adipocyte with brown fat characteristics in vitro.

### EXAMPLE 4. Other artemisinin analogues also promoted browning in addition to artemether

### 4.1 Materials

4.1.1 C3H10T1/2 mesenchymal stem cell line
4.1.2 DMEM medium (Gibco)
4.1.3 Fetal bovine serum (Gibco)
4.1.4 Calf serum (TBD)
4.1.5 Penicillin and streptomycin
2.1.6 Artemether, artemisinin, artesunate, dihydroartemisinin (Chengdu Pufei De Biotech Co., Ltd.)
2.1.7 PVDF membrane (Millipore Immunobilon)
2.1.8 Chemiluminescent No. 5 numECL ultra (NCM Biotech Co., Ltd.) 2.1.9 MitoTracker Green (Life Technology)
2.1.10 Other unmentioned reagents were purchased from Sigma.

### 4.2 Equipment

4.2.1 Constant temperature sterile incubator (Thermo Scientific Forma Series II Water Jacket)
4.2.2 Microscope (Olympus IX711)
4.2.3 Electrophoresis apparatus (BioRad PowerPac HC)
4.2.4 Automatic chemiluminescence apparatus (ImageQuant LAS4000 Mini)

### 4.3 Method

### 4.3.1 Culture and differentiation of C3H10T1/2

According to method 2.4.1.

### 4.3.2 Treatment of compound

Artemisinin, artesunate and dihydroartemisinin were stored in DMSO at a storage concentration of 10mMol/L. The concentration shown in Figure 8 was added on Day 0 of differentiation. Fresh medium was replaced every two days, and the corresponding concentrations of compounds were added until the cells differentiated to mature.

### 4.3.3 Extraction and Western blot of C3H10T1/2 cell total protein

The method for extracting C3H10T1/2 cell total protein was the same as 2.4.3;
Western blot method and catalog number of antibody was the same as 2.4.4.

### 4.4 Results

Compared with control, artemether treated C3H10T1/2 exhibited brown adipocyte-like characteristics: cell was small in size, and had multi-cavity small lipid droplets. C3H10T1/2 treated with artemisinin, artesunate and dihydroartemisinin all exhibited similar morphological characteristics (as shown in Figure 8). Dihydroartemisinin up-regulated the expression of brown fat-related genes.

Dihydroartemisinin (DHA) is a secondary metabolite of artemisinin and its analogues. If dihydroartemisinin has an effect of promoting browning, it means that artemisinin and its analogues all have the effect of promoting browning. After treatment of BMP4-directed C3H10T1/2 with different concentrations (1, 2.5, 5, 7.5 µMol/L) of dihydroartemisinin, the expression levels of brown fat-related genes PGC1α and UCP1 were detected by Western blot, and the expression levels of brown fat-related genes were found to gradually increase with the increase of the concentration of dihydroartemisinin (as shown in Figure 9). The results confirmed that artemisinin analogues had the potential to promote browning of white fat.

### EXAMPLE 5. Artemether inhibited weight gain induced by high-fat diet

### 5.1 Materials

5.1.1 Male C57BL/6J mice (4-6 week) (Shanghai Slike experimental animal Co., Ltd.)
5.1.2 High-fat diet (Research Diets)
5.1.3 Balance (Sartorius)
5.1.4 Artemether (Chengdu Pufei De Biotech Co., Ltd.)

### 5.2 Method

### 5.2.1 Breeding and administration of mice

The mice were breeded in the SPF animal laboratory of the Laboratory Animal Center of Fudan University at a room temperature of 21 °C-23 °C with a 12-hour light/dark cycle. The high-fat diet used to induce obese mice had a fat content of 60%. When the mice were grown to 8-week old, they were randomly divided into a control group and an administration group, with 8 mice in each group. While the mice were fed with a high-fat diet, artemether was injected subcutaneously. Artemether was diluted in PBS at a concentration of 15 µMοl/L, and injected into bilateral inguinal region, with 200 µl being injected into each side. Mice in control group were injected with the same volume of PBS. Administration was continued for eight weeks and the weight of mice was recorded weekly.

### 5.2.2 Data analysis

Data difference was analyzed by Student T test method, and p<0.05 indicates statistical difference.

### 5.3 Results

The weight of mice injected subcutaneously with artemether was significantly lower than that of mice in control group. After six weeks of administration, the difference in weight between the two groups was statistically different, as shown in Figure 10A. The weight gain of mice in administration group was significantly slower than that of mice in control group, as shown in Figure 10B. Subcutaneous injection of artemether significantly inhibited the weight gain of mice under a high-fat diet condition.

### EXAMPLE 6. Subcutaneous injection of artemether reduced adipose tissue in high fat-fed mice

### 6.1 Materials

6.1.1 Male C57BL/6J mice (4-6 week) (Shanghai Slike experimental animal Co., Ltd.)
6.1.2 High-fat diet (Research Diets)
6.1.3 Paraformaldehyde (4%, Sigma)
6.1.4 Artemether (Chengdu Pufei De Biotech Co., Ltd.)

### 6.2 Method

### 6.2.1 Breeding and administration of mice

The mice were breeded in the SPF animal laboratory of the Laboratory Animal Center of Fudan University at a room temperature of 21 °C-23 °C with a 12-hour light/dark cycle. The high-fat diet used to induce obese mice had a fat content of 60%. When the mice were grown to 8-week old, they were randomly divided into a control group and an administration group, with 8 mice in each group. While the mice were fed with a high-fat diet, artemether was injected subcutaneously. Artemether was diluted in PBS at a concentration of 15 µMol/L, and injected into bilateral inguinal region, with 200 µl being injected into each side. Mice in control group were injected with the same volume of PBS. Administration was continued for eight weeks. The mice were sacrificed by cervical dislocation and were dissected, and inguinal fat (Inguinal, subcutaneous fat), epididymal fat (Gonadal, visceral fat) and brown fat in the interscapular region (BAT, classic brown fat) were removed, weighed and fixed. The histological analysis of adipose tissue was commissioned by Shanghai Saige Biotech Co., Ltd.. The histological analysis process included the steps of fixing, paraffin embedding, sectioning and HE staining.

### 6.2.2 Data analysis

Data difference was analyzed by Student T test method, and p<0.05 indicates statistical difference.

### 6.3 Results

Three representative pieces of adipose tissue (inguinal fat, epididymal fat and brown fat) were removed and analyzed. It was found that volume (Figure 11A) and weight (Figure 11B) of adipose tissue in mice injected subcutaneously with artemether was significantly smaller than that of mice in control group. Sectioning and staining of adipose tissue showed that adipocytes of mice in administration group were significantly smaller than that of mice in control group (Figure 11C). Artemether reduced volume and weight of fat in high fat-fed mice, and the weight loss caused by artemether was mainly due to the decrease of adipose tissue.

### EXAMPLE 7. Subcutaneous injection of artemether improved glucose metabolism in obese mice induced by high-fat diet

### 7.1 Materials

7.1.1 Male C57BL/6J mice (4-6 week, Shanghai Slike experimental animal Co., Ltd.)
7.1.2 High-fat diet (Research Diets)
7.1.3 Glucose injection
7.1.4 Insulin for Injection (Humulin)
7.1.5 Glucometer (Roche OneTouch ExtraEasy)
7.1.6 Blood glucose test strips (Roche OneTouch ExtraEasy)
7.1.8 Artemether (Chengdu Pufei De Biotech Co., Ltd.)

### 7.2 Method

### 7.2.1 Breeding and administration of mice

The mice were breeded in the SPF animal laboratory of the Laboratory Animal Center of Fudan University at a room temperature of 21 °C-23 °C with a 12-hour light/dark cycle. The high-fat diet used to induce obese mice had a fat content of 60%. When the mice were grown to 8-week old, they were randomly divided into a control group and an administration group, with 8 mice in each group. While the mice were fed with a high-fat diet, artemether was injected subcutaneously. Artemether was diluted in PBS at a concentration of 15 µMol/L, and injected into bilateral inguinal region, with 200 µl being injected into each side. Mice in control group were injected with the same volume of PBS. Administration was continued for eight weeks.

### 7.2.2 Glucose tolerance test

The mice were fasted for 12-16 hours and injected intraperitoneally with glucose injection at a dose of 2mg/kg. At 0 min, 30 min, 60 min, 90 min, and 120 min after injection, blood was collected from the tail vein, and blood glucose was measured with OneTouch ExtraEasy glucometer and OneTouch ExtraEasy blood glucose test strips and the results were recorded.

### 7.2.3 Insulin tolerance test

Insulin was injected intraperitoneally at a dose of 0.75U/kg weight. At 0 min, 15 min, 30 min, 45 min and 60 min after injection, blood was collected from the tail vein, and blood glucose was measured.

### 7.2.4 Data analysis

Data difference was analyzed by Student T test method, and p<0.05 indicates statistical difference.

### 7.3 Results

Glucose tolerance of mice injected subcutaneously with artemether after eight weeks of high-fat diet feeding was significantly better than that of mice in control group (Figure 12A), and insulin tolerance was significantly better than that of mice in control group (Figure 12B). Under high-fat diet feeding condition and after eight weeks of administration, glucose metabolism and insulin sensitivity of mice injected subcutaneously with artemether were significantly better than that of mice in control group, indicating that artemether played a role in maintaining glucose metabolism homeostasis.

### EXAMPLE 8. Artemether inhibited fatty liver induced by high-fat diet

### 8.1 Materials

8.1.1 Male C57BL/6J mice (4-6 week) (Shanghai Slike experimental animal Co., Ltd.)
8.1.2 High-fat diet (Research Diets)
8.1.3 Artemether (Chengdu Pufei De Biotech Co., Ltd.)

### 8.2 Method

The mice were breeded in the SPF animal laboratory of the Laboratory Animal Center of Fudan University at a room temperature of 21 °C-23 °C with a 12-hour light/dark cycle. The high-fat diet used to induce obese mice had a fat content of 60%. When the mice were grown to 8-week old, they were randomly divided into a control group and an administration group, with 8 mice in each group. While the mice were fed with a high-fat diet, artemether was injected subcutaneously. Artemether was diluted in PBS at a concentration of 15 µMοl/L, and injected into bilateral inguinal region, with 200 µl being injected into each side. Mice in control group were injected with the same volume of PBS. Administration was continued for eight weeks. The mice were sacrificed by cervical dislocation, and liver tissues of mice were removed. The histological analysis including tissue fixation, paraffin embedding, sectioning and HE staining was commissioned by Shanghai Saige Biotech Co., Ltd.

### 8.4 Results

After eight weeks of high-fat diet feeding, obvious lipid accumulation was found in the liver of mice in control group, while only a small amount of lipid was found in the liver of mice injected subcutaneously with artemether. The condition of fatty liver was significantly improved than that of mice in control group. Subcutaneous injection of artemether effectively inhibited fatty liver induced by high-fat diet.

### EXAMPLE 9. Subcutaneous injection of artemether induced browning of subcutaneous fat in high fat-fed mice

### 9.1 Materials

9.1.1 Male C57BL/6J mice (4-6 week, Shanghai Slike experimental animal Co., Ltd.)
9.1.2 High-fat diet (Research Diets)
9.1.3 Mouse thermometer (Physitemp BAT-12)
9.1.4 UCPlantibody (Abcam, ab10983, 1:50)
9.1.5 Artemether (Chengdu Pufei De Biotech Co., Ltd.)

### 9.2 Method

### 9.2.1 Breeding and administration of mice

The mice were breeded in the SPF animal laboratory of the Laboratory Animal Center of Fudan University at a room temperature of 21 °C-23 °C with a 12-hour light/dark cycle. The high-fat diet used to induce obese mice had a fat content of 60%. When the mice were grown to 8-week old, they were randomly divided into a control group and an administration group, with 8 mice in each group. While the mice were fed with a high-fat diet, artemether was injected subcutaneously. Artemether was diluted in PBS at a concentration of 15 µMol/L, and injected into bilateral inguinal region, with 200 µl being injected into each side. Mice in control group were injected with the same volume of PBS. Administration was continued for eight weeks. Mice were fasted and housed in a breeding box at 4 °C. The rectal temperature was measured every 2 hours. The mice were sacrificed by cervical dislocation after 6 hours, and the inguinal fat was removed for histological analysis. The histological analysis including tissue fixation, paraffin embedding, sectioning and immunohistochemistry experiment of UCP1 was commissioned by Shanghai Saige Biotech Co., Ltd.

### 9.2.2 Data analysis

Data difference was analyzed by Student T test method, and p<0.05 indicates statistical difference.

### 9.3 Results

After eight weeks of administration, the body temperature of mice injected subcutaneously with artemether was significantly higher than that of mice in control group when exposed to an environment of 4 °C (Figure 14A). Maintaining body temperature in a cold exposure environment is a physiological function undertaken by thermogenic adipose tissue. The epididymal fat was removed and subjected to immunohistochemical test of UCP1, and the results showed that subcutaneous fat of mice in administration group exhibited significant UCP1 expression (Figure 14B). After eight weeks of subcutaneous administration, mice injected subcutaneously with artemether had a significant increase in ability of maintaining body temperature, and obvious browning was found in subcutaneous fat. It was indicated that artemether inhibited weight gain and improved metabolism by promoting browning of subcutaneous fat.

### EXAMPLE 10. Intraperitoneal injection of artemether inhibited weight gain induced by high-fat diet

### 10.1 Materials

10.1.1 Male C57BL/6J mice (4-6 week)
10.1.2 High-fat diet (Research Diets)
10.1.3 Balance (Sartorius)
10.1.4 Artemether (Wuhan Feierde Biotech Co., Ltd.)
10.1.5 Coconut oil (Aladdin)

### 10.2 Method

### 10.2.1 Breeding and administration of mice

The mice were breeded in the SPF animal laboratory of the Laboratory Animal Center of Fudan University at a room temperature of 21 °C-23 °C with a 12-hour light/dark cycle. The high-fat diet used to induce obese mice had a fat content of 60%. When the mice were grown to 8-week old, they were randomly divided into a control group and an administration group, with 8 mice in each group. While the mice were fed with a high-fat diet, artemether was injected intraperitoneally. Artemether for intraperitoneal injection was stored at 4 °C in coconut oil at a storage concentration of 6mg/ml (20mMol/L), and protected from light. Mice in administration group were injected with artemether at a dose of 20mg/kg weight. Administration was twice a week for eight weeks, and the weight of the mice was recorded weekly.

### 10.2.2 Data analysis

Data difference was analyzed by Student T test method, and p<0.05 indicates statistical difference.

### 10.3 Results

The weight of mice injected intraperitoneally with artemether was significantly lower than that of mice in control group. After three weeks of administration, the difference in weight between the two groups was statistically different, as shown in Figure 15A. The weight gain of mice in administration group was significantly slower than that of mice in control group, as shown in Figure 15B. Intraperitoneal injection of artemether significantly inhibited the weight gain induced by high-fat diet.

### EXAMPLE 11. Intraperitoneal injection of artemether significantly reduced adipose tissue in high fat-fed mice

### 11.1 Materials

11.1.1 Male C57BL/6J mice (4-6 week, Shanghai Slike experimental animal Co., Ltd.)
11.1.2 High-fat diet (Research Diets)
11.1.3 Paraformaldehyde (4%, Sigma)
11.1.4 Artemether (Chengdu Pufei De Biotech Co., Ltd.)
11.1.5 Coconut oil (Aladdin)

### 11.2 Method

### 11.2.1 Breeding and administration of mice

The mice were breeded in the SPF animal laboratory of the Laboratory Animal Center of Fudan University at a room temperature of 21 °C-23 °C with a 12-hour light/dark cycle. The high-fat diet used to induce obese mice had a fat content of 60%. When the mice were grown to 8-week old, they were randomly divided into a control group and an administration group, with 8 mice in each group. While the mice were fed with a high-fat diet, artemether was injected intraperitoneally. Artemether for intraperitoneal injection was stored at 4 °C in coconut oil at a storage concentration of 6mg/ml (20mMol/L), and protected from light. Mice in administration group were injected with artemether at a dose of 20mg/kg weight. Administration was twice a week for eight weeks. The mice were sacrificed by cervical dislocation and were dissected, and inguinal fat (Inguinal, subcutaneous fat), epididymal fat (Gonadal, visceral fat) and brown fat in the interscapular region (BAT, classic brown fat) were removed, weighed and fixed. The histological analysis of adipose tissue was commissioned by Shanghai Saige Biotech Co., Ltd., which included the steps of fixing, paraffin embedding, sectioning and HE staining.

### 11.2.2 Data analysis

Data difference was analyzed by Student T test method, and p<0.05 indicates statistical difference.

### 11.3 Results

Three representative pieces of adipose tissue were removed and analyzed. It was found that volume (Figure 16A) and weight (Figure 16B) of adipose tissue in mice injected subcutaneously with artemether was significantly smaller than that of mice in control group. Sectioning and staining of adipose tissue showed that adipocytes of mice in administration group were significantly smaller than that of mice in control group (Figure 16C). Intraperitoneal injection of artemether effectively inhibited fat accumulation caused by a high-fat diet.

### EXAMPLE 12. Intraperitoneal injection of artemether improved glucose metabolism in obese mice induced by high-fat diet

### 12.1 Materials

12.1.1 Male C57BL/6J mice (4-6 week, Slike experimental animal Co., Ltd.)
12.1.2 High-fat diet (Research Diets)
12.1.3 Artemether (Chengdu Pufei De Biotech Co., Ltd.)
12.1.4 Coconut oil(Aladdin)
12.1.5 Glucose injection
12.1.6 Insulin for Injection (Humulin)
12.1.7 Glucometer (Roche OneTouch ExtraEasy)
12.1.8 Blood glucose test strips (Roche OneTouch ExtraEasy)
12.1.9 Chloral hydrate (10%, Sigma)
12.1.10 Automatic biochemical analyzer and calibrator for blood glucose Cfas and kit (Roche)

### 12.2 Method

### 12.2.1 Breeding and administration of mice

The mice were breeded in the SPF animal laboratory of the Laboratory Animal Center of Fudan University at a room temperature of 21 °C-23 °C with a 12-hour light/dark cycle. The high-fat diet used to induce obese mice had a fat content of 60%. When the mice were grown to 8-week old, they were randomly divided into a control group and an administration group, with 8 mice in each group. While the mice were fed with a high-fat diet, artemether was injected intraperitoneally. Artemether for intraperitoneal injection was stored at 4 °C in coconut oil at a storage concentration of 6mg/ml (20mMol/L), and protected from light. Mice in administration group were injected with artemether at a dose of 20mg/kg weight. Administration was twice a week for eight weeks.

### 12.2.2 Glucose tolerance test

The mice were fasted for 12-16 hours and injected intraperitoneally with glucose injection at a dose of 2mg/kg weight. At 0 min, 30 min, 60 min, 90 min, and 120 min after injection, blood was collected from the tail vein, and blood glucose was measured with OneTouch ExtraEasy glucometer and OneTouch ExtraEasy blood glucose test strips and the results were recorded.

### 12.2.3 Insulin tolerance test

Insulin was injected intraperitoneally at a dose of 0.75U/kg weight. At 0 min, 15 min, 30 min, 45 min and 60 min after injection, blood was collected from the tail vein, and blood glucose was measured.

### 12.2.4 Monitoring of random blood glucose

The mice were anesthetized with 10% chloral hydrate and blood was taken from the orbit. The whole blood was centrifuged at 300 rpm for 5 min at 4 °C. Serum was taken and the blood glucose levels were measured by automatic biochemical analyzer and calibrator for blood glucose Cfas and kit (Roche).

### 12.2.5 Data analysis

Data difference was analyzed by Student T test method, and p<0.05 indicates statistical difference.

### 12.3 Results

Random blood glucose of mice injected intraperitoneally with artemether after eight weeks of high-fat diet feeding was significantly lower than that of mice in control group, glucose tolerance was significantly better than that of mice in control group (Figure 12A), and insulin sensitivity was also significantly better than that of mice in control group (Figure 12B). Intraperitoneal injection of artemether effectively reduced blood glucose in high-fat diet-fed obese mice, and significantly improved glucose metabolism and insulin sensitivity.

### EXAMPLE 13. Intraperitoneal injection of artemether effectively inhibited fatty liver in obese mice induced by high-fat diet

### 13.1 Materials

13.1.1 Male C57BL/6J mice (4-6 week) (Shanghai Slike experimental animal Co., Ltd.)
13.1.2 High-fat diet (Research Diets)
13.1.3 Artemether (Chengdu Pufei De Biotech Co., Ltd.)
13.1.4 Coconut oil (Aladdin)

### 13.2 Method

The mice were breeded in the SPF animal laboratory of the Laboratory Animal Center of Fudan University at a room temperature of 21 °C-23 °C with a 12-hour light/dark cycle. The high-fat diet used to induce obese mice had a fat content of 60%. When the mice were grown to 8-week old, they were randomly divided into a control group and an administration group, with 8 mice in each group. While the mice were fed with a high-fat diet, artemether was injected intraperitoneally. Artemether for intraperitoneal injection was stored at 4 °C in coconut oil at a storage concentration of 6mg/ml (20mMol/L), and protected from light. Mice in administration group were injected with artemether at a dose of 20mg/kg weight. Administration was twice a week for eight weeks. The mice were sacrificed by cervical dislocation, and liver tissues of mice were removed and fixed. The histological analysis for liver was commissioned by Shanghai Saige Biotech Co., Ltd., and the histological analysis process included tissue fixation, paraffin embedding, sectioning and HE staining.

### 13.4 Results

After eight weeks of high-fat diet feeding, obvious lipid accumulation was found in the liver of mice in control group, while only a small amount of lipid was found in the liver of mice injected intraperitoneally with artemether, and the morphology of the liver cells was almost normal, as shown in Figure 18. Intraperitoneal injection of artemether effectively inhibited fatty liver induced by high-fat diet.

### EXAMPLE 14. Iintraperitoneal injection of artemether inhibited elevated blood lipid caused by high-fat diet

### 14.1 Materials

14.1.1 Male C57BL/6J (4-6 week) mice (Slike experimental animal Co., Ltd.)
14.1.2 High-fat diet (Research Diets)
14.1.3 Artemether (Chengdu Pufei De Biotech Co., Ltd.)
14.1.4 Coconut oil(Aladdin)
14.1.5 Chloral hydrate (10%, Sigma)
14.1.6 Lipid calibrator Cfas Lipid and other calibrators Cfas used in automatic biochemical analyzer, and detection kits for related indicators (Roche)

### 14.2 Method

### 14.2.1 Breeding and administration of mice

The mice were breeded in the SPF animal laboratory of the Laboratory Animal Center of Fudan University at a room temperature of 21 °C-23 °C with a 12-hour light/dark cycle. The high-fat diet used to induce obese mice had a fat content of 60%. When the mice were grown to 8-week old, they were randomly divided into a control group and an administration group, with 8 mice in each group. While the mice were fed with a high-fat diet, artemether was injected intraperitoneally. Artemether for intraperitoneal injection was stored at 4 °C in coconut oil at a storage concentration of 6mg/ml (20mMol/L), and protected from light. Mice in administration group were injected with artemether at a dose of 20mg/kg weight. Administration was twice a week for eight weeks.

### 14.2.2 Detection of serum indicators in mice

The mice were anesthetized with 10% chloral hydrate and blood was taken from the orbit. The whole blood was centrifuged at 300 rpm for 5 min at 4 °C. Serum was taken and the serum indicators were measured by lipid calibrator Cfas Lipid, high-density lipoprotein Cfas, and low density lipoprotein Cfas, as well as detection kits for related indicators.

### 14.2.3 Data analysis

Data difference was analyzed by Student T test method, and p<0.05 indicates statistical difference.

### 14.3 Results

After eight weeks of high-fat diet feeding, triglyceride and high-density lipoprotein (HDL) in the serum of mice injected intraperitoneally with artemether were not different from the control group. Serum cholesterol and low density lipoprotein (LDL) were significantly decreased compared with the control group, as shown in Figure 19. Artemether significantly improved dyslipidemia caused by a high-fat diet.

### EXAMPLE 15. Intraperitoneal injection of artemether induced browning of subcutaneous fat in high fat-fed mice

### 15.1 Materials

15.1.1 Male C57BL/6J mice (4-6 week)
15.1.2 High-fat diet (Research Diets)
15.1.3 Mice thermometer (Physitemp BAT-12)
15.1.4 UCP1-specific antibody (Abcam, ab10983, 1:50)
15.1.5 Artemether (Chengdu Pufei De Biotech Co., Ltd.)
15.1.6 Coconut oil (Aladdin)

### 15.2 Method

### 15.2.1 Breeding and administration of mice

The mice were breeded in the SPF animal laboratory of the Laboratory Animal Center of Fudan University at a room temperature of 21 °C-23 °C with a 12-hour light/dark cycle. The high-fat diet used to induce obese mice had a fat content of 60%. When the mice were grown to 8-week old, they were randomly divided into a control group and an administration group, with 8 mice in each group. While the mice were fed with a high-fat diet, artemether was injected intraperitoneally. Artemether for intraperitoneal injection was stored at 4 °C in coconut oil at a storage concentration of 6mg/ml (20mMol/L), and protected from light. Mice in administration group were injected with artemether at a dose of 20mg/kg weight. Administration was twice a week for eight weeks. Mice were fasted and housed in a breeding box at 4 °C. The rectal temperature was measured every 2 hours. The mice were sacrificed by cervical dislocation after 6 hours, and the inguinal fat was removed for histological analysis. The histological analysis including tissue fixation, paraffin embedding, sectioning and immunohistochemistry experiment of UCP1 was commissioned by Shanghai Saige Biotech Co., Ltd.

### 15.2.2 Data analysis

Data difference was analyzed by Student T test method, and p<0.05 indicates statistical difference.

### 15.3 Results

After eight weeks of administration, the body temperature of mice injected intraperitoneally with artemether was significantly higher than that of mice in control group when exposed to an environment of 4 °C (Figure 19A). Maintaining body temperature in a cold exposure environment was a physiological function undertaken by thermogenic adipose tissue. The epididymal fat of mice was removed and subjected to immunohistochemical test of UCP1, and the results showed that subcutaneous fat of mice in administration group exhibited significant UCP1 expression (Figure 19B). After eight weeks of intraperitoneal injection of artemether, mice in administration group had a significant increase in ability of maintaining body temperature, and obvious browning was found in subcutaneous fat. It was indicated that artemether inhibited weight gain by promoting browning of subcutaneous fat.

### EXAMPLE 16. Artemether inhibited weight gain in diabetic mice ob/ob mice

### 16.1 Materials

16.1.1 Male ob/ob mice (4 week, Model Animal Research Center of Nanjing University)
16.1.2 Balance (Sartorius)
16.1.3 Artemether (Chengdu Pufei De Biotech Co., Ltd.)
16.1.4 Coconut oil (Aladdin)

### 16.2 Method

### 16.2.1 Breeding and administration of mice

The ob/ob mice were breeded in the SPF animal laboratory of the Laboratory Animal Center of Fudan University at a room temperature of 21 °C-23 °C with a 12-hour light/dark cycle. When the mice were grown to 4-week old, they were randomly divided into a control group and an administration group, with 4 mice in each group. The administration group was injected intraperitoneally with artemether. Artemether for intraperitoneal injection was stored at 4 °C in coconut oil at a storage concentration of 6mg/ml (20mMol/L), and protected from light. Ob/ob mice in administration group were injected with artemether at a dose of 20mg/kg weight. Administration was twice a week for eight weeks, and the weight of the mice was recorded weekly.

### 16.2.3 Data analysis

Data difference was analyzed by Student T test method, and p<0.05 indicates statistical difference.

### 16.3 Results

The weight of ob/ob mice in administration group tended to be lower than that of mice in control group, but was not statistically different (Figure 21A). However, the weight gain of the mice in administration group was significantly slower than that of mice in control group (Figure 21B), indicating that artemether significantly inhibited the weight gain of ob/ob mice. However, the difference between the administration group and the control group was not statistically significant at the time of administration for eight weeks. Artemether effectively inhibited the weight gain of diabetic animal model ob/ob mice.

### EXAMPLE 17. Artemether improved fatty liver in diabetic ob/ob mice

### 17.1 Materials

17.1.1 Male ob/ob mice (4-week, Model Animal Research Center of Nanjing University)
17.1.2 Artemether (Wuhan Feierde Biotech Co., Ltd.)
17.1.3 Coconut oil (Aladdin)

### 17.2 Method

Ob/ob mice were breeded in the SPF animal laboratory of the Laboratory Animal Center of Fudan University at a room temperature of 21 °C-23 °C with a 12-hour light/dark cycle. When the mice were grown to 4-week old, they were randomly divided into a control group and an administration group, with 4 mice in each group. Mice in administration group were injected intraperitoneally with artemether. Artemether for intraperitoneal injection was stored at 4 °C in coconut oil at a storage concentration of 6mg/ml (20mMol/L), and protected from light. Ob/ob mice in administration group were injected with artemether at a dose of 20mg/kg weight. Administration was twice a week for eight weeks. The mice were sacrificed by cervical dislocation, and liver tissues of mice were removed, fixed and paraffin embedded. The histological analysis was commissioned by Shanghai Saige Biotech Co., Ltd..

### 17.3 Results

The fatty liver of ob/ob mice in artemether administration group was significantly relieved compared with that of mice in control group (Figure 22); artemether effectively relieved the fatty liver of diabetic animal model ob/ob.

### EXAMPLE 18. Intraperitoneal injection of artemether did not affect the growth of healthy mice

### 18.1 Materials

18.1.1 Male C57BL/6J mice (4-6 week)
18.1.2 Normal diet (Shanghai Slike experimental animal Co., Ltd.)
18.1.3 Balance (Satorius)
18.1.4 Artemether (Wuhan Feierde Biotech Co., Ltd.)
18.1.5 Coconut oil(Aladdin)

### 18.2 Method

### 18.2.1 Breeding and administration of mice

The mice were breeded in the SPF animal laboratory of the Laboratory Animal Center of Fudan University at a room temperature of 21 °C-23 °C with a 12-hour light/dark cycle. When the mice were grown to 8-week old, they were randomly divided into a control group and an administration group, with 8 mice in each group. Mice in administration group were injected intraperitoneally with artemether. Artemether for intraperitoneal injection was stored at 4 °C in coconut oil at a storage concentration of 6mg/ml (20mMol/L), and protected from light. Mice in administration group were injected with artemether at a dose of 20mg/kg weight. Mice in control group were injected with 100µl coconut oil. Administration was twice a week for eight weeks and the weight of the mice was recorded weekly.

### 18.2.2 Data analysis

Data difference was analyzed by Student T test method, and p<0.05 indicates statistical difference.

### 18.3 Results

There was no difference in the weight of mice in administration group and control group during the eight weeks of administration (Figure 23A). The weight gain of mice in administration group was also not significantly different from that of mice in control group (Figure 23B). Intraperitoneal injection of artemether did not affect the normal growth of healthy lean mice.

### EXAMPLE 19. Intraperitoneal injection of artemether did not affect glucose metabolism in healthy lean mice

### 19.1 Materials

19.1.1 Male C57BL/6J mice (4-6 week, Slike experimental animal Co., Ltd.)
19.1.2 Normal diet (Shanghai Slike experimental animal Co., Ltd.)
19.1.3 Glucose injection
19.1.4 Insulin for Injection (Humulin)
19.1.5 Glucometer (Roche OneTouch ExtraEasy)
19.1.6 Blood glucose test strips (Roche OneTouch ExtraEasy)

### 19.2 Method

### 19.2.1 Breeding and administration of mice

The mice were breeded in the SPF animal laboratory of the Laboratory Animal Center of Fudan University at a room temperature of 21 °C-23 °C with a 12-hour light/dark cycle. When the mice were grown to 8-week old, they were randomly divided into a control group and an administration group, with 8 mice in each group. Artemether for intraperitoneal injection was stored at 4 °C in coconut oil at a storage concentration of 6mg/ml (20mMol/L), and protected from light. Mice in administration group were injected with artemether at a dose of 20mg/kg weight, and mice in control group were injected with 100µl coconut oil. Administration was twice a week for eight weeks.

### 19.2.2 Glucose tolerance test

The mice were fasted for 12-16 hours and injected intraperitoneally with glucose injection at a dose of 2mg/kg. At 0 min, 30 min, 60 min, 90 min, and 120 min after injection, blood was collected from the tail vein, and blood glucose was measured with OneTouch ExtraEasy glucometer and OneTouch ExtraEasy blood glucose test strips and the results were recorded.

### 19.2.3 Insulin tolerance test

Insulin was injected intraperitoneally at a dose of 0.75U/kg weight. At 0 min, 15 min, 30 min, 45 min and 60 min after injection, blood was collected from the tail vein, and blood glucose was measured.

### 19.2.4 Data analysis

Data difference was analyzed by Student T test method, and p<0.05 indicates statistical difference.

### 19.3 Results

Healthy lean mice were injected intraperitoneally with artemether for eight weeks. The glucose tolerance curve of mice in administration group did not differ from that of mice in control group, indicating that glucose metabolism was not affected by artemether, as shown in Figure 23A. The insulin tolerance curve of mice in administration group also did not differ from that of mice in control group, as shown in Figure 24B; intraperitoneal injection of artemether did not alter glucose metabolism of healthy lean mice.

### EXAMPLE 20. Intraperitoneal injection of 20 mg/kg weight of artemether did not cause liver damage in mice

### 20.1 Materials

20.1.1 Male C57BL/6J mice (4-6 week)
20.1.2 Normal diet (Shanghai Slike experimental animal Co., Ltd.)
20.1.3 Artemether (Wuhan Feierde Biotech Co., Ltd.)
20.1.4 Coconut oil (Aladdin)

### 20.2 Method

The mice were breeded in the SPF animal laboratory of the Laboratory Animal Center of Fudan University at a room temperature of 21 °C-23 °C with a 12-hour light/dark cycle. When the mice were grown to 8-week old, they were randomly divided into a control group and an administration group, with 8 mice in each group. Mice in administration group were injected intraperitoneally with artemether. Artemether for intraperitoneal injection was stored at 4 °C in coconut oil at a storage concentration of 6mg/ml (20mMol/L), and protected from light. Mice in administration group were injected with artemether at a dose of 20mg/kg weight. Mice in control group were injected 100µl coconut oil. Administration was twice a week for eight weeks. The mice were sacrificed by cervical dislocation, and liver tissues of mice were removed and fixed. Liver tissue fixation, paraffin embedding, sectioning and HE staining was commissioned by Shanghai Saige Biotech Co., Ltd..

### 20.3 Results

The morphology of liver cells of healthy lean mice injected intraperitoneally with artemether for eight weeks was normal, and had no difference from that of mice in control group, as shown in Figure 25; in the case of intraperitoneal injection of artemether at a dose of 20 mg/kg weight twice a week for eight weeks, artemether had no hepatotoxicity.

### EXAMPLE 21. Artemether did not alter food intake of mice

### 21.1 Materials

21.1.1 Male C57BL/6J mice (4-6 week, Shanghai Slike experimental animal Co., Ltd.)
21.1.2 Normal diet (Shanghai Slike experimental animal Co., Ltd.)
21.1.3 High-fat diet (Research Diets)
21.1.4 Artemether (Wuhan Feierde Biotech Co., Ltd.)
21.1.5 Coconut oil (Aladdin)

### 21.2 Method

### 21.2.1 Breeding and administration of mice

The mice were breeded in the SPF animal laboratory of the Laboratory Animal Center of Fudan University at a room temperature of 21 °C-23 °C with a 12-hour light/dark cycle. When the mice were grown to 8-week old, they were randomly divided into four groups, a high-fat control group, a high-fat administration group, a normal administration group, and a normal control group, with 8 mice in each group. Administration groups (high-fat and normal diet) were injected intraperitoneally with artemether. Artemether for intraperitoneal injection was stored at 4 °C in coconut oil at a storage concentration of 6mg/ml (20mMol/L), and protected from light. Mice in administration group were injected with artemether at a dose of 20mg/kg weight, and mice in control group were injected with 100µl coconut oil. The diet was weighed weekly and the diet consumption of mice in each group was recorded.

### 21.2.2 Data analysis

Data difference was analyzed by Student T test method, and p<0.05 indicates statistical difference.

### 21.3 Results

For high-fat groups, the diet consumption of mice in administration group was substantially the same as that of mice in control group with no difference, as shown in Figure 26A. For normal groups, the diet consumption of mice in administration group was not different from that of mice in control group, as shown in Figure 26B. Under the conditions of both normal diet and high fat diet, intraperitoneal injection of artemether did not affect normal food intake in mice. Under the conditions of this example, artemether did not cause anorexia nervosa.

## Claims

1. Use of an artemisinin analogue represented by the following general formula (A) in the preparation of a medicament for promoting lipolysis and/or preventing or treating a metabolism-related disease: 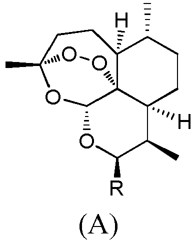 wherein, R is selected from hydroxyl, methoxy, ethoxy, butanedioic acid monoester group (succinate group), 2-aminoethoxy, 2R-3-tert-butylamino-2-hydroxypropoxy, thiomorpholinyl and 1,1-dioxidothiomorpholinyl.

2. A method for promoting lipolysis and/or preventing or treating a metabolism-related disease, **characterized in that** the method comprises administering to a subject in need thereof a therapeutically effective amount of an artemisinin analogue represented by the following general formula (A): 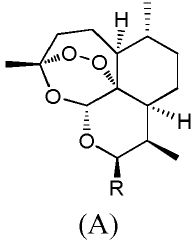 wherein, R is selected from hydroxyl, methoxy, ethoxy, butanedioic acid monoester group (succinate group), 2-aminoethoxy, 2R-3-tert-butylamino-2-hydroxypropoxy, thiomorpholinyl and 1,1-dioxidothiomorpholinyl.

3. The method according to claim 2, **characterized in that**, said administering a therapeutically effective amount of said artemisinin analogue is by oral or injection; preferably, the administration dosage of said artemisinin analogue is 20mg/kg-100mg/kg, preferably 50mg/kg-100mg/kg for oral administration, and 10mg/kg-50mg/kg, preferably 20mg/kg-40mg/kg for injection, respectively.

4. An artemisinin analogue for promoting lipolysis and/or preventing or treating a metabolism-related disease, **characterized in that**, said artemisinin analogue is represented by the following general formula (A): 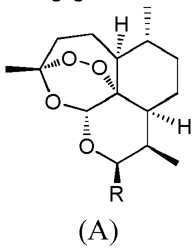 wherein, R is selected from hydroxyl, methoxy, ethoxy, butanedioic acid monoester group (succinate group), 2-aminoethoxy, 2R-3-tert-butylamino-2-hydroxypropoxy, thiomorpholinyl and 1,1-dioxidothiomorpholinyl.

5. The use, artemisinin analogue or method according to any of the preceding claims, **characterized in that**, said artemisinin analogue is artemether represented by formula (II):

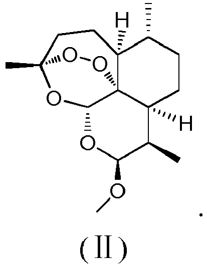

6. The use, artemisinin analogue or method according to any one of claims 1 to 5, **characterized in that**, said promoting lipolysis comprises promoting browning of white fat.

7. The use, artemisinin analogue or method according to any one of claims 1 to 5, **characterized in that**, said promoting lipolysis and/or preventing or treating a metabolism-related disease comprises controlling weight and improving metabolism.

8. The use, artemisinin analogue or method according to any one of claims 1 to 5, **characterized in that**, said metabolism-related disease is hyperglycemia, insulin resistance, dyslipidemia, and/or fatty liver caused by obesity.

9. A pharmaceutical composition for promoting lipolysis and/or preventing or treating a metabolism-related disease, **characterized in that**, said pharmaceutical composition comprises: (1) the artemisinin analogue according to any one of claims 4 to 5, and (2) a pharmaceutically acceptable carrier.

10. The method according to claim 9, **characterized in that**, preferably, the dosage form of said pharmaceutical composition for oral administration is selected from an oral suspension, a powder and a granule; and the dosage form of said pharmaceutical composition for injection is selected from an aqueous solution, an oil and a suspension.
